# EUROPÄISCHE PATENTSCHRIFT

(11) **EP 1 599 194 B1**
(45) Veröffentlichungstag und Bekanntmachung des Hinweises auf die Patenterteilung: **17.11.2010**
(21) Anmeldenummer: 04700037.7
(22) Anmeldetag: 02.01.2004
(51) Int. Cl.: A61K 31/60, A61P 31/12

(54) **VERWENDUNG VON ACETYLSALIZILSÄURE ZUR PROPHYLAXE UND/ODER THERAPIE VON VIRUSERKRANKUNGEN**
USE OF ACETYLSALICYLIC ACID FOR THE PROPHYLAXIS AND/OR THERAPY OF VIRAL DISEASES
UTILISATION DE L'ACIDE ACETYLSALICYLIQUE DESTINEES A LA PROPHYLAXIE ET/OU LA THERAPIE DE MALADIES VIRALES

(30) Priorität: 03.01.2003 DE 10300222
(43) Veröffentlichungstag der Anmeldung: 30.11.2005
(60) Teilanmeldung: 10011214.3
(73) Patentinhaber: Activaero GmbH, 35285 Gemünden (DE)
(72) Erfinder: PLANZ, Oliver, 72108 Rottenburf (DE); PLESCHKA, Stephan, 35390 Giessen (DE); SEDLACEK, Hans-Harald, 35041 Marburg (DE); LUDWIG, Stephan, 48149 Münster (DE)
(74) Vertreter: Vossius & Partner
(86) Internationale Anmeldenummer: PCT/DE2004/000012
(87) Internationale Veröffentlichungsnummer: WO 2004/060360

(56) Entgegenhaltungen:
- EP-A- 0 417 385
- WO-A-00/50633
- WO-A-01/83547
- WO-A-98/52540
- WO-A-02/051413
- DE-A- 3 832 799
- GB-A- 2 374 008
- US-A- 6 107 281
- US-A- 6 130 226
- US-B1- 6 514 955
- PRIMACHE V ET AL: "In vitro activity of acetylsalicylic acid on replication of varicella-zoster virus." THE NEW MICROBIOLOGICA : OFFICIAL JOURNAL OF THE ITALIAN SOCIETY FOR MEDICAL, ODONTOIATRIC, AND CLINICAL MICROBIOLOGY (SIMMOC). OCT 1998, Bd. 21, Nr. 4, Oktober 1998 (1998-10), Seiten 397-401, XP009031179 ISSN: 1121-7138
- BROGGINI M ET AL: "Flurbiprofen versus ASA in influenza symptomatology: a double-blind study." INTERNATIONAL JOURNAL OF CLINICAL PHARMACOLOGY RESEARCH. 1986, Bd. 6, Nr. 6, 1986, Seiten 485-488, XP009031176 ISSN: 0251-1649
- INGLOT A D: "Comparison of the antiviral activity in vitro of some non-steroidal anti-inflammatory drugs." THE JOURNAL OF GENERAL VIROLOGY. MAR 1969, Bd. 4, Nr. 2, März 1969 (1969-03), Seiten 203-214, XP009031196 ISSN: 0022-1317
- BETTINI R ET AL: "Diclofenac sodium versus acetylsalicylic acid: a randomized study in febrile patients." THE JOURNAL OF INTERNATIONAL MEDICAL RESEARCH. 1986, Bd. 14, Nr. 2, 1986, Seiten 95-100, XP009031192 ISSN: 0300-0605
- YOUNKIN S W ET AL: "Reduction in fever and symptoms in young adults with influenza A/Brazil/78 H1N1 infection after treatment with aspirin or amantadine." ANTIMICROBIAL AGENTS AND CHEMOTHERAPY. APR 1983, Bd. 23, Nr. 4, April 1983 (1983-04), Seiten 577-582, XP009031195 ISSN: 0066-4804
- BERNASCONI P ET AL: "Evaluation of a new pharmaceutical form of nimesulide for the treatment of influenza." DRUGS UNDER EXPERIMENTAL AND CLINICAL RESEARCH. 1985, Bd. 11, Nr. 10, 1985, Seiten 739-743, XP009031193 ISSN: 0378-6501
- MILVIO C: "TREATMENT OF INFLUENZA SYNDROME A DOUBLE-BLIND CONTROLLED TRIAL OF NIMESULIDE VS. ASPIRIN" CLINICAL TRIALS JOURNAL, Bd. 22, Nr. 1, 1985, Seiten 111-117, XP009031194 ISSN: 0009-9325
- HUANG R T ET AL: "Anti-influenza viral activity of aspirin in cell culture." THE NEW ENGLAND JOURNAL OF MEDICINE. 22 SEP 1988, Bd. 319, Nr. 12, 22. September 1988 (1988-09-22), Seite 797, XP009031184 ISSN: 0028-4793 in der Anmeldung erwähnt
- COX N J N J ET AL: "Influenza" LANCET, XX, XX, Bd. 354, Nr. 9186, 9. Oktober 1999 (1999-10-09), Seiten 1277-1282, XP004262683 ISSN: 0140-6736
- MONTO A S: "The role of antivirals in the control of influenza" VACCINE, BUTTERWORTH SCIENTIFIC. GUILDFORD, GB, Bd. 21, Nr. 16, 1. Mai 2003 (2003-05-01), Seiten 1796-1800, XP004418060 ISSN: 0264-410X
- YU K-L ET AL: "Novel quinolizidine salicylamide influenza fusion inhibitors" BIOORGANIC & MEDICINAL CHEMISTRY LETTERS, OXFORD, GB, Bd. 9, Nr. 15, 2. August 1999 (1999-08-02), Seiten 2177-2180, XP004174154 ISSN: 0960-894X
- YIN M-J ET AL: "THE ANTI-INFLAMMATORY AGENTS ASPIRIN AND SALICYLATE INHIBIT THE ACTIVITY OF IKAPPAB KINASE-BETA" NATURE, MACMILLAN JOURNALS LTD. LONDON, GB, Bd. 396, 5. November 1998 (1998-11-05), Seiten 77-80, XP002938591 ISSN: 0028-0836 in der Anmeldung erwähnt
- MAY M J ET AL: "Selective inhibition of NF-kB activation by a peptide that blocks the interaction of NEMO with the IkB kinase complex" SCIENCE, AMERICAN ASSOCIATION FOR THE ADVANCEMENT OF SCIENCE,, US, Bd. 289, 1. September 2000 (2000-09-01), Seiten 1550-1554, XP002189523 ISSN: 0036-8075 in der Anmeldung erwähnt
- CHU WEN-MING ET AL: "JNK2 and IKKbeta are required for activating the innate response to viral infection" IMMUNITY, Bd. 11, Nr. 6, Dezember 1999 (1999-12), Seiten 721-731, XP002282727 ISSN: 1074-7613 in der Anmeldung erwähnt

## Beschreibung

### Gebiet der Erfindung.

Die Erfindung betrifft eine pharmazeutische Zusammensetzung enthaltend Acetylsalicylsäure (ASS) zur Behandlung viraler Infekte.

### Stand der Technik und Hintergrund der Erfindung

Infektionen mit RNA- oder DNA-Viren stellen eine bedeutende Gesundheitsbedrohung für Mensch und Tier dar. Zu den RNA-Viren gehören die Negativ-Strang-RNA Viren, wie beispielsweise Influenza Viren oder das Borna-Disease Virus. Infektionen mit Influenza-Viren gehören immer noch zu den großen Seuchen der Menschheit und fordern Jahr für Jahr eine Vielzahl an Todesopfern. Sie sind gesamtwirtschaftlich, etwa durch krankheitsbedingte Arbeitsunfähigkeit, ein immenser Kostenfaktor. Von ebenfalls wichtiger wirtschaftlicher Bedeutung sind Infektionen mit dem Borna Disease Virus (BDV), das vor allem Pferde und Schafe befällt, jedoch auch schon beim Menschen isoliert wurde und hier in Zusammenhang mit neurologischen Erkrankungen gebracht wird.

Das Problem der Bekämpfung von insbesondere RNA-Viren ist die durch eine hohe Fehlerrate der viralen Polymerasen verursachte wandlungsfähigkeit der Viren, die sowohl die Herstellung geeigneter Impfstoffe als auch das Entwickeln von antiviralen Substanzen sehr schwierig macht.

Darüber hinaus hat sich gezeigt, dass die Anwendung von antiviralen Substanzen, die direkt gegen Funktionen des Virus gerichtet sind, zwar zu Therapiebeginn eine gute antivirale Wirkung zeigen, aber mutationsbedingt sehr schnell zur Selektion resistenter Varianten führt. Ein Beispiel hierfür ist das anti-Influenza-Agens Amantadin und dessen Derivate, welches bzw. welche gegen ein Transmembranprotein des Virus gerichtet ist bzw. sind. Innerhalb kurzer Zeit nach Anwendung bilden sich resistente Varianten des Virus.

Ein weiteres Beispiel sind die neuen Therapeutika für Influenzainfektionen, die das Influenza-virale Oberflächenprotein Neuraminidase hemmen. Hierzu gehört beispielsweise Relanza. In Patienten wurden bereits Relanza-resistente Varianten gefunden (Gubareva et al., J Infect Dis 178, 1257-1262, 1998). Die Hoffnungen, die in dieses Therapeutikum gelegt wurden, konnten somit nicht erfüllt werden.

Aufgrund ihrer meist sehr kleinen Genome und damit verbundener begrenzter Kodierungskapazität für replikationsnotwendige Funktionen sind alle Viren in starkem Maße auf Funktionen ihrer Wirtszellen angewiesen. Durch Einflussnahme auf solche zelluläre Funktionen, die für die virale Replikation notwendig sind, ist es möglich, die Virusreplikation in der infizierten Zelle negativ zu beeinträchtigen. Hierbei besteht für das Virus nicht die Möglichkeit, durch Anpassung, insbesondere durch Mutationen, die fehlende zelluläre Funktion zu ersetzen, um so dem Selektionsdruck zu entweichen. Dies konnte am Beispiel des Influenza A Virus mit relativ unspezifischen Hemmstoffen gegen zelluläre Kinasen und Methyltransferasen bereits gezeigt werden (Scholtissek und Müller, Arch Virol 119, 111-118, 1991).

Es ist bekannt, dass Zellen über eine Vielzahl von Signalübertragungswegen verfügen, mit Hilfe derer auf die Zelle einwirkende Signale in den Zellkern übertragen werden. Dadurch kann die Zelle auf äußere Reize reagieren und mit Zellproliferation, Zellaktivierung, Differenzierung oder kontrolliertem Zelltod reagieren.

Diesen Signalübertragungswegen ist gemeinsam, dass sie mindestens eine Kinase enthalten, welche durch Phosphorylierung mindestens ein nachfolgend signalübertragendes Protein aktivieren.

Bei Betrachtung der zellulären Prozesse, die nach Virusinfektionen induziert werden, findet man, dass eine Vielzahl von DNA-und RNA-Viren in der infizierten Wirtszelle bevorzugt einen definierten Signalübertragungsweg, den sogenannte Raf/MEK/ERK-Kinase-Signalübertragungsweg aktivieren. Dieser Signalübertragungsweg ist einer der wichtigsten Signalübertragungswege in einer Zelle und spielt eine bedeutende Rolle in Proliferations- und Differenzierungsprozessen (Cohen, Trends in Cell Biol 7, 353-361, 1997; Robinson und Cobb, Curr.Opin.Cell Biol 9, 180-186, 1997; Treisman, Curr.Opin Cell Biol 8, 205-215, 1996).

Die Untersuchung der Rolle dieses Signalübertragungsweges in zellulären Entscheidungsprozessen hat zur Identifizierung mehrerer pharmakologischer Inhibitoren geführt, welche den Signalübertragungsweg unter anderem auf der Ebene von MEK, also relativ am Beginn des Signalübertragungsweges hemmen (Alessi et al., J Biol Chem 270, 27489-27494, 1995; Cohen, Trends in Cell Biol 7, 353-361, 1997; Dudley et al., PNAS USA 92, 7686-7689, 1995; Favata et al., J Biol Chem 273, 18623-18632, 1998).

Neuere Daten zeigen, dass die Inhibition des Ras-Raf-MEK-ERK-Signalübertragungsweges oder eines weiteren Signalübertragungsweges, des MEKK/SEK/JNK-Signalübertragungsweges, durch Wirksubstanzen, welche relativ selektiv eine der an diesem Signalübertragungsweg beteiligten Kinasen, beispielsweise die MEK oder die SEK inhibieren, die intrazelluläre Vermehrung von intranukleär replizierenden Negativstrang-Viren, beispielsweise von Influenza A Virus und Borna Disease Virus (BDV) drastisch hemmen kann (Pleschka et al., Nature Cell Biol 3, 301-305, 2001; Planz et al., J Virol 10, 4871-4877, 2001; PCT/DE 01/01292; DE 101 38 912).

Bislang war bekannt, dass Influenza- Viren vorzugsweise den Raf-MEK-ERK-Signalübertragungsweg oder den MEKK/SEK Signalübertragungsweg für ihre Vermehrung nutzen und demzufolge eine Hemmung dieser Signalübertragungswege zu einer vollständigen Hemmung der Virusvermehrung führt. Da in einer Zelle die Signalübertragungswege jedoch kaum eine in sich abgeschlossene Funktionen aufweisen, sondern bei Aktivierung des einen Signalübertragungsweges über Kreuzvernetzungen weitere Signalübertragungswege in unterschiedlichem Ausmaß zusätzlich aktiviert werden, ist grundsätzlich nicht auszuschließen, dass ein gehemmter Signalübertragungsweg sowohl von der Zelle als auch von den Viren umgangen werden kann und der therapeutische Effekt einer Wirksubstanz, die eine Virusvermehrung durch Hemmung eines bestimmten Signalübertragungsweges hemmt, hierdurch eingeschränkt werden könnte.

Daher besteht ein großer Bedarf für das Auffinden und Verwenden antiviraler Wirksubstanzen, welche zusätzlich oder ergänzend zu solchen wirken, die Kinasen von zellulären Signalübertragungswegen, beispielsweise des Raf-MEK-ERK-Signalübertragungsweges oder des MEKK/SEK/JNK-Signalübertragungsweges hemmen. Solche Wirkstoffe sind bereits in den Literaturstellen PCT/DE 01/01292 und DE 101 38 912 beschrieben worden.

Einer der bedeutenden Signalübertragungswege in der Zelle ist der nukleäre Faktor von kappaB (NF-kB)-Signalübertragungs- weg. Zentraler Bestandteil dieses Übertragungsweges ist der heterodimere NF-kB-Transkriptions-Proteinkomplex, bestehend einerseits aus p50 [entstanden aus der Proteolyse von NF-kB1 (p105)] oder aus p52 [entstanden aus der Proteolyse von NF-kappaB2 (p100)] und andererseits aus p65 (RELA), c-REL oder RELB. Der häufigste NF-kB-Komplex besteht aus p50 und p65.

Die Transkriptionsaktivität dieses NF-kB-Komplexes wird gehemmt durch die Bindung der Inhibitorproteine von NFkB (IkB) an die nukleäre Bindesequenz von p65. Hierdurch verbleibt der Komplex im Zytoplasma. Aktivierung der Zelle beispielsweise durch Wachstumsfaktoren, Chemokine, TNFalpha, Il-1, CD40-ligand, LPS oder im Rahmen einer Infektion mit Viren führt zur Aktivierung von Kinasen wie der "NF-kB-induzierenden Kinase" (NIK), der Kinase TAK, der Kinase AKT und möglicherweise auch der Kinase MEKK1. Diese Kinase führen zur Aktivierung des "Inhibitor von kB (IkB)-Kinase" (IKK)-Komplexes, bestehend aus IKKalpha, IKKbeta und NEMO. Aktiviertes IKK phosphoryliert IkB und führt so zu dessen Degradierung und erlaubt damit die Freisetzung, nukleäre Translokation und Aktivierung der Transskriptionsaktivität des p50/p65- Heterodimers. Ein weiterer NF-kB Komplex besteht aus p100 und RELB. Aktivierung der Zelle durch Lymphotoxine führt, vermutlich bevorzugt vermittelt durch NIK, zur Aktivierung von IKKalpha. Das aktivierte IKK induziert die Phosphorylierung und damit die Proteolyse von p100 zum p52, welches wiederum im Komplex mit dem RELB in den Zellkern translozieren und dort als Transkriptionsfaktor aktiv werden kann.

Es ist bekannt, dass i) nicht-steroidale anti-inflammatorische Substanzen (NSAIDs), wie beispielsweise Sulindac (Yamamoto et al., J Biol Chem 274,27307-27314, 1999; Berman et al., Clin Cancer Res 8, 354-360, 2002) oder Derivate von Sulindac wie beispielsweise Sulindac Sulfoxid, Sulidac Sulfon, Sulindac Sulfid oder Benzylamid Sulindac-Analoga (Moon und Lerner, Cancer Research 62, 5711-5719,2002) oder Acetylsalizylsäure oder Salizylsäure (Yin et al., Nature 396, 77-80,1998) oder Curcumin (Oncogene 18, 6013-6020, 1999) durch Hemmung der IKKbeta, ii) NEMO bindende Peptide (May et al., Science 289, 1550-1554, 2000), Proteosom Inhibitoren wie beispielsweise PS-341 (Tan und Waldmann, Cancer Res 62, 1083-1086,2002; Adams Trends Mol Med 8, 49-54, 2002) oder iii) Antisense Nukleotidsequenzen spezifisch für p65 oder p50 (Higgins et al., PNAS-USA 90, 9901-9905,1993) die Aktivierung des NF-kB-Signalübertragungsweges hemmen können. Bislang wurden diese Hemmstoffe jedoch ausnahmslos geprüft für ihre Verwendung als Wirkstoffe zur Beeinflussung von Entzündungen und des Wachstums von Tumoren, da bei beiden Indikationen die beteiligten Zellen eine erhöhte Aktivierung des NF-kB Signalübertragungsweg aufweisen (Karin et al., Nature Reviews Cancer 2, 301-310, 2002).

Bislang bestand die Vorstellung, dass eine Virusinfektion, im Besonderen auch eine Infektion mit Influenza-Virus, über die Aktivierung der IKK den NF-kB Signalweg aktiviert, welcher wiederum entscheidend beteiligt ist an der durch diese Infektion erhöhten Expression von antiviral aktiven Proteinen wie beispielsweise Interferon (Chu et al., Immunity 11,721-731,1999). In Übereinstimmung mit dieser Vorstellung ist der Befund, dass die Influenza-Virus induzierte Aktivität des Interferon-β-Promotors stark reduziert ist in Zellen, welche eine transdominant negative Mutation von IKK2 oder von IkBalpha exprimieren (Wang et al., Virol 74,11566-11573,2000). Andererseits gibt es den diesen Vorstellungen und Befunden widersprechenden Hinweis, dass Acetylsalizylsäure, bekannt als Inhibitor der IKK , in der Zellkultur auch Influenza-Virus Infektionen hemmen kann, dieses jedoch erst ab Konzentrationen von 5-10 mM (entsprechend 0,9-1,8 mg/ml). Derartige Konzentrationen sind im Blut nach oraler Verabreichung von Acetylsalizylsäure ohne erhebliche Nebenwirkungen praktisch nicht erreichbar (Huang und Dietsch, New Engl J Med 319,797, 1988). Acetylsalizylsäure gilt als das toxischste aller derzeit frei verfügbare Analgetika mit der geringsten therapeutischen Breite (Jones, Am J Ther 9, 295-257,2002). Zwar spekulierten Huang und Dietsch, durch eine Verabreichung von Aerosol höhere und damit antiviral wirksame Konzentrationen lokal in den Atemwegen zu erzielen. Diese Empfehlungen wurden bislang jedoch nicht umgesetzt, da allein schon die bisher bekannten klinischen Nebenwirkungen nach oraler Verabreichung einer ansonsten als nicht-toxisch geltenden Dosis von 100mg Acetylsalizylsäure dazu führten, vor der Einnahme von Aspirin bei Virusgrippe besonders bei Kindern zu warnen (Gebrauchsinformation Z.Nr.14.252 der Bayer AG zum Acetylsalicylsäure (ASS)-Präparat Aspirin®)

### Technisches Problem der Erfindung

Der Erfindung liegt das technische Problem zu Grunde, eine Formulierung mit ASS zur Behandlung viraler Infektionen anzugeben.

### Grundzüge der Erfindung und Ausführungsformen.

Die Erfindung beruht auf den Erkenntnissen, dass i) Wirkstoffe, welche den zellulären NF-kB Signalübertragungsweg hemmen, die Vermehrung von Viren in einem Organismus hemmen können, ii) bei lokaler Verabreichung geringere Konzentrationen des erfindungsgemäß eingesetzten Wirkstoffes antiviral wirksam sind als aus den in vitro Daten ableitbar, iii) der erfindungsgemäß eingesetzte Wirkstoff Acetylsalizylsäure aerogen in Konzentrationen von 0.1 bis 4 mM verabreicht eine deutliche Hemmung der Influenza-Virusvermehrung in der Lunge und im Gesamtorganismus und einen systemischen Heileffekt ohne Beeinträchtigung des Allgemeinbefindens bewirkt, obwohl derartige Konzentrationen der Acetylsalizylsäure in vitro keine antivirale Wirksamkeit gezeigt haben. Höhere Dosen von Acetylsalizylsäure, beispielsweise Acetylsalizylsäure in Konzentrationen von 10 mM, waren vergleichsweise nicht stärker, noch höhere Dosen (bis 50 mM) deutlich geringer antiviral wirksam als die niedrigen, erfindungsgemäßen Konzentrationen. Zusätzlich erwiesen sich Konzentrationen ab 20mM nach aerogener Verabreichung als toxisch.

Gegenstand der vorliegenden Erfindung ist somit die Verwendung gemäß Anspruch 1. Bestandteil des NF-kB Signalübertragungs- weges sind beispielsweise: Tumor necrosis factor receptor associated factor (TRAF), NF-kB inducing kinase (NIK), Mitogen-activated protein kinase kinase Kinase 1 (MEKKK1), Mitogen-activated protein kinase kinase kinase 3 (MEKKK3), AKR mouse thymoma Kinase (AKT), TGFβ activated kinase (TAK1), Inhibitor of NF-kB kinase alpha (IKKalpha), Inhibitor of NF-kB kinase beta (IKKbeta), NEMO, Inhibitor von kB (IkB), RELA (p65), C-REL, RELB, NF-kB1 (p105), NF-kB2 (p100), P50, P52.

Bevorzugt erfolgt die erfindungsgemäße Verwendung zur Prophylaxe oder Therapie einer Viruserkrankungen, die durch RNA- oder DNA-Viren, vorzugsweise Negativstrang-RNA Viren, beispielsweise Influenza-Viren, oder Borna-Viren, hervorgerufen werden.

Weiterhin bevorzugt ist die Verwendung von Acetylsalizylsäure zur Prophylaxe oder Therapie einer Influenza-Virus-Infektion, wobei die Acetylsalizylsäure bronchial (aerogen) in Konzentrationen von vorzugsweise 0,1 bis 4 mM verabreicht wird. Je nach Anwendung kann die Untergrenze aber auch zwischen 0,1 und 20 mg bzw. 50 mg liegen. Je nach Anwendung kann aber auch die Obergrenze zwischen 1 mg bzw. 2 mg und den angegebenen Maximalwerten liegen. Eine Tagesdosis wird vorteilhafterweise in 1 bis 8 Gaben verabreicht, welche zweckmäßigerweise gleichmäßig über eine Wachdauer von 16 Stunden verteilt sind. Eine Behandlung erfolgt zweckmäßigerweise über einen Zeitraum von 1 bis 7 Tagen und länger. Die Erfindung umfaßt insofern auch galenisch hergerichtet Gabeeinheiten, wobei die in einer Gabeeinheit vorliegende Menge des Wirkstoffes gemäß vorstehenden Bereichen eines Behandlungsplanes unschwer errechenbar ist.

Eine weitere Ausführungsform der vorliegenden Erfindung betrifft ein Kombinationspräparat gemäß einem der Ansprüche 3 oder 4. Zu den weiteren antiviral wirkenden Wirksubstanzen in dem erfindungsgemäßen Kombinationspräparat gehören entweder mindestens eine andere erfindungsgemäße Wirksubstanz und/oder mindestens eine antvirale Wirksubstanz wie beispielsweise: Amantadin (1-Adamantanamin) und dessen Derivate, welches bzw. welche gegen ein Transmembranprotein von einigen Influenza-A Viren gerichtet ist bzw. sind, wie beispielsweise das Rimantadine, Therapeutika für Influenzainfektionen, die das Influenza-virale Oberflächenprotein Neuraminidase hemmen. Hierzu gehört beispielsweise Relanza, Inhibitoren des Raf-MEK-ERK- Signalübertragungsweges wie zum Beispiel U0126 oder weiterer Inhibitoren, wie sie in der PCT/DE 01/01292 beschrieben wurden, Inhibitoren des MEKK/SEK-Signalübertragungsweges oder der Komponenten weiterer Signalübertragungswege wie sie in der DE 10138 912 beschrieben wurden und synthetische Nucleosidanaloga wie beispielweise 3-Deaza adenosin und Ribavirin.

Das Kombinationspräparat kann in Form eines Gemisches oder als einzelne Komponenten zur gleichzeitigen oder zeitlich unterschiedlichen Anwendung an gleichen oder unterschiedlichen Orten systemisch oder lokal angewendet werden.

Die Verabreichung des Kombinationspräparates kann als Mischung der Wirksubstanzen erfolgen. Die Wirksubstanzen können pro Verabreichung jedoch auch getrennt voneinander an dem gleichen Ort, oder auch an voneinander getrennten Orten, gleichzeitig oder auch zu unterschiedlichen Zeitpunkten innerhalb eines Zeitraumes, in welchem die zuerst verabreichte Substanz noch Wirkung zeigt, beispielsweise in einem Zeitraum von drei Tagen, verabreicht werden.

Die erfindungsgemäße Verabreichung der Wirksubstanz ist die aerogene, das heißt bronchiale Verabreichung der Wirksubstanz zur Prophylaxe oder Therapie derjenigen Virusinfektionen, welche aerogen infizieren. Hierzu werden galenische Hilfsmittel und Zerstäuber der Wirksubstanz verwendet, wie sie dem Fachmann hinreichend bekannt sind.

Geeignete Hilfs- und Zusatzstoffe, die beispielsweise der Stabilisierung oder Konservierung des Arzneimittels oder Diagnostikums dienen, sind dem Fachmann allgemein bekannt (siehe z. B. Sucker H et al., (1991) Pharmazeutische Technologie, 2. Auflage, Georg Thieme Verlag, Stuttgart). Beispiele für solche Hilfs- und/oder Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Die Wirksubstanz wird unter sterilen Bedingungen, mit einem physiologisch akzeptablen Trägerstoff und möglichen Preservativen, Puffern oder Treibmitteln, je nach Bedarf, vermischt. Derartige Trägerstoffe für Arzneimittelzubereitungen sind dem Fachmann geläufig.

Bevorzugt wird die erfindungsgemäße Wirksubstanz in einer einmaligen Dosis, besonders bevorzugt in mehreren Dosen verabreicht, wobei die einzelnen Dosen die maximal tolerable Dosis (MTD) der Wirksubstanz für den Menschen nicht übersteigt. Vorzugsweise wird eine Dosis gewählt, welche die Hälfte der MTD beträgt. Die Tagesdosis kann einmalig am Tag oder in mehreren Teilportionen aufgeteilt über den Tag hinweg, vorzugsweise in etwa gleichen Zeitabständen verabreicht werden.

### Beispiel 1: Testsystem zur Auffindung von antiviralen Wirkstoffen

Mit Hilfe retroviraler Transduktion der humanen Lungenepithelzellen A549, der caninen Nierenepitehlzellen MDCK und der Affenzellen Vero wurden Zelllinien hergestellt, welche stabil entweder eine dominant negative Form der IKK (IKK KD) oder eine dominant-interferierende mutierte Form des Inhibitors von NF-kB, mIkB, exprimieren. Darüber hinaus wurden ebenfalls entsprechende Linien generiert, welche eine aktive Form der IKK (IKK EE) exprimieren. Sowohl die Konstrukte als auch Ihre Wirksamkeit auf die NF-kB vermittelte Genexpression in Zellen wurden bereits beschrieben (Denk et al J Biol Chem 276, 28451-28458, 2001).

Zur Generierung der stabilen Zelllinen wurde die entsprechenden cDNAs für IKK KD, mIkB und IKK EE in sense-Orientierung in den retroviralen Expressionsvektor pCFG5 IEGZ (Kuss et al. Eur J Immunol 29,3077-3088,1999) einkloniert. Neben der Boten-RNA des gene of interest' kodiert die Vektor DNA noch für die Boten-RNA des "green fluorescent protein" (GFP), welches während der Proteinsynthese ausgehend von einer internen Ribosomenbindestelle exprimiert wird. Dies erlaubt die Identifizierung stabil transduzierter Zellen in der Durchflusszytometrie. Darüber hinaus vermittelt der Vektor eine Resistenz gegen das Antibiotikum Zeöcin. Die verschiedenen Expressionskonstrukte sowie der Leervektor wurden mit Hilfe der Calciumphosphatprezipitationsmethode in die virusproduzierende Zelline ∅NX (Grignani et al., Cancer Res 58,14-19,1998) transfiziert (Denk et al., J Biol Chem 276, 28451-28458, 2001). Die Transfektionseffizienz wurde nach 24h anhand der GFP Expression kontrolliert und war in der Größenordnung von 70-80%. Die Zellen wurden darauf hin für ca. 2 Wochen mit 1mg/ml Zeocin im Medium selektioniert.

Zur Infektion der verschiedenen Zielzellen (A549, MDCK, Vero) mit den rekombinanten Retroviren wurden die retrovirushaltigen Mediumüberstände der virusproduzierenden Zellinien filtriert, mit 5 µg/ml Polybrene (Sigma) versetzt und auf frische Zellen gegeben. Die Infektion erfolgte während 2mal 3-stündiger Zentrifugation (1000xg) an zwei aufeinander folgenden Tagen. Stabil transduzierte Zellen wurden 24h nach Infektion für zwei weitere Wochen mit 400-600 µg/ml Zeozin im Mediumüberstand selektioniert. Nach der so erfolgten Generierung der stabilen Zellinien wurden sowohl diese als auch Wildtyp Influenza A Virus wie im folgenden beschrieben infiziert und die Virustiter von Zellen, welche stabil den Vektor, IKK KD, mIkB und IKK EE trugen im Vergleich zum Titer aus dem Überstand von Wildtyp Zellen bestimmt.

Parallel wurden MDCK Zellen mit Hilfe des Transfektionsreagenz Lipofectamine 2000 (Life Technologies) nach Standardmethoden (Ludwig et al., J Biol Chem 276,10990-10998, 2001) mit den gleichen Konstrukten transient transfiziert. Die Transfektionseffizienzen lagen bei >60%. 24h nach Transfektion erfolgte wie bei den stabilen Linien Infektion mit dem Influenza A Virusstamm fowl plaque virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft. Verglichen wurde auch hier der Virustiter von Influenza A Virus-infizierten Zellen, welche entweder mit dem Leervektor oder Konstrukten welche IKK KD, mIkB und IKK EE exprimierten, transfiziert waren.

Es zeigten sich die folgenden Ergebnisse. Der Vergleich der Virustiter von Influenza A Virus-infizierten MDCK Zellen, welche zuvor entweder mit dem Leervektor oder einem Konstrukt, welches IKK KD oder mIkB exprimierte, transfiziert worden waren, zeigte, dass in IKK KD oder mIkB exprimierenden Zellen die Vermehrung der Viren nach 24h zu 50%-70% gehemmt war. Entsprechend fand man in Zellen, welche die aktive Form von IKK, IKK EE exprimierten, ein Steigerung der Virusvermehrung. Diese Ergebnisse waren in mehreren unabhängigen Ansätzen reproduzierbar. Entsprechende Ergebnisse konnten auch in den stabilen A549, MDCK und Vero Zelllinien erhalten werden, welche entweder IKK KD, mIkB oder IKK EE exprimierten, wobei die größten Effekte in stabil transfizierten A549 Zellen beobachtet wurden. Hier führte eine Hemmung der NF-kB Aktivierung durch IKK KD oder mIkB zu einer bis zu 10-fachen Reduktion der Virustiter, während konstitutive Aktivierung des Signalwegs durch stabile Expression von IKK EE die Virusausbeute um das zehnfache verstärkte. Diese Befunde zeigen, dass Aktivierung von IKK und NF-kB essentiell für die Influenza Virus Vermehrung ist und die spezifische Inhibition des IKK/NF-kB Signalmoduls s zu einer signifikanten Reduktion der Virusproduktion führt.

### Beispiel 2: Hemmung der viralen NF-kB Aktivierung und Reduktion der Influenza Virus in vitro mit Acetylsalizylsäure (ASS)

Salicylate, wie ASS oder Sulfosalazine finden weitgehende klinische Anwendung als Schmerzmittel und antiinflammatorische Agenzien. Neuere Publikationen zeigen, dass diese Substanzen direkte und effektive Inhibitoren der IKK sind (Yin et al., Nature 396, 77-80, 1998; Weber et al., Gastroenterology 119,1209-1218, 2000) wie auch in vitro die Vermehrung von Influenzaviren hemmen können (Huang und Dietsch, New Engl J Med 319,797,1988 ). Als Positivkontrolle diente somit ASS. A549 Lungenepithelzellen wurden mit ansteigenden Konzentrationen von ASS im Bereich 0,01 mM-5mM behandelt. Diese Konzentrationen verblieben über das gesamte Experiment im Kulturmedium. Eine Stunde nach Behandlung erfolgte Infektion mit dem Influenza A Virusstamm fowl plaque virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft.

In einem zweiten Ansatz wurden MDCK Zellen ein Stunde vor Infektion bzw. zwei und vier Stunden nach Infektion mit 5 mM ASS behandelt. Infektion und Nachweis der neu-gebildeten Viren erfolgte wie oben.

Es zeigten sich die folgenden Ergebnisse. Nach Vergleich der Virustiter aus A549 Kulturüberständen 24h p.i. von nicht ASS-behandelten mit ASS-behandelten Zellen ergab sich ein konzentrationsabhängige Inhibition der Virusvermehrung, die jedoch erst deutlich war bei 5mM und hier 2 log Stufen umfasste. Eine entsprechende hemmende Wirkung (> 2 log Stufen) von 5mM ASS konnte auch bei infizierten MDCK Zellen beobachtet werden, wobei hier bei Zugabe von ASS 4h nach Infektion immer noch eine Reduktion der Virustiter um das 10-fache bewirkte.

### Beispiel 3: Wirkung von ASS auf die Influenzainfektion in der Maus

### Aerogene Verabreichung

C57 Bl/6 Mäuse wurden mit 5000 pfu/20µl bzw mit 10.000 pfu/20 ml Influenza-Virus (Fowl Plague Virus ,FPV) nasal infiziert. Bei Behandlungsbeginn am Tag 0 wurde 30 min nach der Infektion in die Mäuse unter Betäubung (i.p. Injektion von 300µl Ketamin/Rompun; Serum Werk Bernburg; Bayer Ag Leverkusen) ein Tracheotubus eingeführt und mit Hilfe eines Nebulizers (Hugo Sachs Elektronik-Harvard App.GmbH March-Hugstetten) Aerosol (Verabreichung von 600 µl) enthaltend 2mM (= 0,36mg/ml PBS), 10 mM, 20mM oder 50 mM ASS (Sigma-Aldrich Steinheim) oder zur Kontrolle nur PBS verabreicht. Diese aerogene Verabreichung der Prüfsubstanzen wurde in einigen Gruppen an den Tagen 1, 2, 3 oder 1, 2, 5 und 6 wiederholt. In weiteren Gruppen wurde die Behandlung mit ASS an den Tagen 3, 4, 5, 6 nach Infektion durchgeführt. Pro Gruppe wurden 5-6 Tiere behandelt. Körpergewicht, Todesrate und Überlebensdauer wurde bestimmt. In einem weiteren Versuch wurden nach Behandlung mit ASS am Tag 1 am Tag 3 die Tiere getötet und die Viruskonzentration in dem Lungengewebe bestimmt.

Es zeigten sich die folgenden Ergebnisse. Geringere Dosen von ASS reduzierten die Viren in der Lunge deutlicher als hohe Dosen. ASS erwies sich ab 20 mM als toxisch (Abnahme des Körpergewichtes). Während in der Kontrollgruppe alle Tiere an Influenza starben, überlebten in den Gruppen behandelt mit niedrigen nichttoxischen Dosen (2mM) von ASS etwa 40% die Infektion. Des weiteren war die mittlere Überlebenszeit der verstorbenen Tiere nach ASS im Vergleich zur Kontrollgruppe deutlich verlängert.

Die Ergebnisse in vivo Experimente gemäß diesem Beispiel zeigen, dass die einfache oder mehrfache (bis zu 3x) tägliche aerogene Verabreichung von ASS in niedriger nichtoxischer Dosis, i.e. in Dosen von 0,1 mg/kg bis 300 mg/kg Körpergewicht, insbesondere 10 mg/kg bis 100 mg/kg, vorzugsweise 20 mg/kg bis 50 mg/kg, beispielsweise 30 mg/kg, zu einer deutlichen therapeutischen Wirkung gegen eine tödliche durch nasale Verabreichung des Influenza-Virus induzierte Erkrankung führt. Dabei sollte die Formulierung so gewählt sein, dass die aerogen zu verabreichende pharmazeutische Zusammensetzung ASS in Konzentrationen unterhalb 2 mM, vorzugsweise 0,01 mM bis 1,99 mM, höchstvorzugsweise 0,1 mM oder 0,5 mM bis 1,5 mM, beispielsweise 1 mM liegt. Die Flüssigphasenmenge an Aerosol ist dabei entsprechend der oben angegebenen Tagesdosen unter Berücksichtigung der eingesetzten Konzentration in der Flüssigphase auszurechnen und einzurichten. Letzteres läßt sich beispielsweise mit üblichen Zerstäubungsvorrichtungen erreichen, welche definierte Mengen einer Lösung zu einem Aerosol versprühen.

## Patentansprüche

1. Verwendung von Acetylsalizylsäure (ASS) zur Herstellung einer pharmazeutischen Zusammensetzung zur Prophylaxe und/oder Therapie einer Viruserkrankung, wobei eine Komponente des NF-kB Signalübertragungsweges derart gehemmt wird, dass eine Virusvermehrung gehemmt wird, wobei die pharmazeutische Zusammensetzung zur bronchialen Darreichung als Aerosol hergerichtet ist, und wobei die Tagesdosis, bezogen auf ASS, beim Menschen im Bereich von 0,1 bis 70 mg liegt.

2. Pharmazeutische Zusammensetzung zur Prophylaxe und/oder Therapie einer Viruserkrankung enthaltend ASS, wobei der Wirkstoff aerogen in Konzentrationen von 0,1 bis 4 mM verabreicht wird.

3. Ausführungsform nach Anspruch 1 oder 2, wobei die Viruserkrankung durch Infektion mit RNA- oder DNA-Viren, vorzugsweise Influenza-Viren, hervorgerufen wird.

4. Ausführungsform nach einem der Ansprüche 1 bis 3, wobei die pharmazeutische Zusammensetzung zusätzlich eine Wirksubstanz aus "Sulindac, Sulindac-Sulfoxid, Sulindac-Sulfon, Sulindac-Sulfid, Benzylamid Sulindac-Analoga, Salicylsäure, Salicylamid, Salacetamid, Ethenzamid, Diflunisal, Olsalazin oder Salazosulfapyridin, Curcumin, Pyrrolidine-dithiocarbamate (PDTC), Oxicame wie beispielsweise Piroxicam, Penthamethyl-hydroxychroman (PMC), 17 beta Oestradiol, Polyphenole des Tees, wie beispielsweise (-) - epigallocatechin-3-gallat (EGCG), Bay11-7182, PS-341, Lactacystin, und antisense Nukleotidsequenzen spezifisch für p65 oder p50" enthält.

5. Ausführungsform nach Anspruch 4, bei welcher die Wirksubstanz 1-Adamantanamin, Rimantadine, ein Neuraminidaseinhibitor oder ein Nukleosidanalogon, wie Ribavirin, ist.

## Claims

1. Use of acetylsalicylic acid (ASS) for the preparation of a pharmaceutical composition for the prophylaxis and/or treatment of a viral disease, wherein one component of the Nf-kB signal transduction pathway is inhibited such that a virus multiplication is inhibited, wherein the pharmaceutical composition is formulated for bronchial administration by way of an aerosol and wherein the daily dosis for humans of ASS is in the range of 0.1 to 70 mg.

2. Pharmaceutical composition for the prophylaxis and/or treatment of a viral disease containing ASS, wherein the active ingredient is administered by way of an aerosol in concentrations of 0.1 to 4 mM.

3. The embodiment according to claim 1 or 2, wherein the viral disease is caused by an infection by RNA or DNA viruses, preferably influenza viruses.

4. The embodiment according to any one of claims 1 to 3, wherein the pharmaceutical composition additionally comprises an active ingredient selected from sulindac, sulindac sulfoxide, sulindac sulfone, sulindac sulfide, benzylamide sulindac analogues, salicylic acid, salicylamide, salacetamide, ethenzamide, diflunisal, olsalazine or salazosulfapyridine, curcumin, pyrrolidine dithiocarbamate (PDTC), oxicams such as piroxicam, pentamethyl hydroxychromane (PMC), 17 beta oestradiol, polyphenols of tea, such as (-)-epigallo-catechin-3-gallate (EGCG), Bay11-7182, PS-341, lactacystine and antisense nucleotide sequences specific to p65 or p50.

5. The embodiment according to claim 4, wherein the active ingredient is 1-adamantanamine, rimantadine, a neuraminidase inhibitor or a nucleoside analogue such as ribavirin.

## Revendications

1. Utilisation d'acide acétylsalicylique (ASS) pour la fabrication d'une composition pharmaceutique destinée à la prophylaxie et/ou à la thérapie d'une affection virale, où un composant de la voie de transmission de signal NF-kB est inhibé de manière à empêcher une reproduction virale, où la composition pharmaceutique se présente comme aérosol destiné à une administration bronchique, et où la dose journalière rapportée à l'ASS est comprise entre 0,1 et 70 mg pour l'homme.

2. Composition pharmaceutique destinée à la prophylaxie et/ou à la thérapie d'une affection virale contenant de l'ASS, où la substance active est administrée par voie aérogène à une concentration comprise entre 0,1 et 4 mM.

3. Forme d'exécution selon la revendication 1 ou 2, où l'affection virale est provoquée par une infection par des virus à ARN ou à ADN, préférentiellement des virus de la grippe.

4. Forme d'exécution selon l'une des revendications 1 à 3, où la composition pharmaceutique contient en outre une des substances actives suivantes : « sulindac, sulfoxyde de sulindac, sulfone de sulindac, sulfure de sulindac, analogues benzylamides du sulindac, acide salicylique, amide salicylique, salacétamide, éthenzamide, diflunisal, olsalazine ou salazosulfapyridine, curcumine, pyrrolidine dithiocarbamate (PDTC), oxicams tels que piroxicam, penthaméthyl-hydroxychromane (PMC), 17 béta oestradiol, polyphénols du thé tels que (-) - épigallocatéchine-3-gallate (EGCG), Bay11-7182, PS-341, lactacystine, et séquences de nucléotides antisens spécifiques pour p65 ou p50 ».

5. Forme d'exécution selon la revendication 4, où la substance active est : la 1-adamantanamine, la rimantadine, un inhibiteur de la neuraminidase ou un analogue nucléosidique tel que la ribavirine.
